# EUROPEAN PATENT SPECIFICATION

(11) **EP 2 318 482 B1**
(45) Date of publication and mention of the grant of the patent: **08.08.2018**
(21) Application number: 09808863.6
(22) Date of filing: 21.08.2009
(51) Int. Cl.: C07C 7/167, C07C 11/04, B01J 37/20, B01J 35/00, B01J 23/46

(54) **PROCESS TO PURIFY ETHYLENE-CONTAINING OFF-GAS FEED STREAMS**
VERFAHREN ZUR REINIGUNG ETHYLENHALTIGER ABGASSTRÖME
PROCÉDÉ POUR PURIFIER DES COURANTS D'ALIMENTATION EN GAZ DE DÉGAGEMENT CONTENANT DE L'ÉTHYLÈNE

(30) Priority: 21.08.2008 US 195678
(43) Date of publication of application: 11.05.2011
(73) Proprietor: Clariant Corporation, Charlotte, NC 28205 (US)
(72) Inventor: SUN, Mingyong, Louisville KY 40241 (US); BRYAN, Marty, Prospect KY 40059 (US); BLANKENSHIP, Steve, Radcliff KY 40160 (US); URBANCIC, Michael, A., Louisville KY 40241 (US)
(74) Representative: FRKelly
(86) International application number: PCT/US2009/054592
(87) International publication number: WO 2010/022318

(56) References cited:
- WO-A1-98/40450
- GB-A- 879 209
- KR-A- 20000 076 130
- US-A1- 2001 000 035
- US-B1- 6 395 952
- US-B1- 6 395 952

## Description

### TECHNICAL FIELD

The present development is a method that can be useful in purifying raw gas or off-gas streams from steam crackers or fluid catalytic crackers (FCC). By the method of the present development, acetylene, methylacetylene, nitrogen oxides, and oxygen are simultaneously removed from a raw gas feed stream that comprises ethylene, hydrogen, and CO without significant loss of ethylene, using a supported Ruthenium-based catalyst. The catalyst may comprise between 0.01 wt. % to 5 wt. % ruthenium distributed on an alumina support.

### BACKGROUND ART

Catalytic cracking processes, such as fluid catalytic cracking (FCC) and deep catalytic cracking (DCC), have been widely used in industry for many years to produce transportation fuels, such as gasoline and diesel. The off-gases from the FCC and DCC processes contain valuable products such as ethylene and propylene. However, these off-gas streams contain relatively dilute concentrations of olefins and it is generally perceived as not being economically feasible to recover the olefins by conventional means, such as fractionation. Thus, most refineries use the off-gas as fuel gas.

Recently, the recovery of these relatively high value olefins from off-gas streams has gained increasing interest. For example, U.S. Patent 5,981,818 describes a process for recovery of dilute olefins from off-gases. Besides valuable olefins, FCC / DCC off-gases also contain detrimental impurities such as acetylenes and di-olefins. These impurities need to be removed from the off-gas streams in order to utilize the high value olefins in downstream processes. Typically, acetylenes and dienes found in olefin streams are commercially removed by a selective hydrogenation process.

Most selective acetylene hydrogenation operations at the commercial scale use Palladium-based catalysts. In addition to hydrocarbons, an off-gas stream often contains nitrogen oxides, oxygen, sulfur, and other impurities. The Pd-based catalysts have high activity and selectivity for selective hydrogenation of acetylene and dienes; but they are very sensitive to sulfur and some other poisons. Moreover, the Pd-based catalysts are not known to be particularly effective for removal of nitrogen oxides and / or oxygen.

Nickel catalysts have also been used in selective hydrogenation of acetylene and dienes. Nickel catalysts are resistant to sulfur poisoning, but are not selective toward hydrogenation of acetylene. Most commonly, while acetylene is removed, significant amounts of olefins are also hydrogenated to saturated hydrocarbons. Nickel-based catalysts also tend to form nickel carbonyl when the carbon monoxide level is high in the feed gas stream, particularly at low temperatures. Nickel carbonyl is a highly volatile, highly toxic substance that can deposit in downstream equipment and pose a significant safety hazard to workers in the area.

U.S. Patent 2,747,970 teaches and claims a process of removing carbon monoxide and carbon dioxide from a gas stream using a catalyst consisting of 0.01 wt. % to 2.0 wt. % ruthenium on an activated earth metal oxide, such as activated alumina. The process comprises directly contacting the gas stream with the supported catalyst while maintaining a reaction temperature of at least 120°C until the carbon content of the CO and CO₂ is substantially completely converted to methane. However, the process does not teach that the same catalyst and method can be used to remove acetylene, methylacetylene, butadiene, NO, and O₂ from an ethylene gas stream without risk of loss of ethylene. The prior art which does teach the use of ruthenium catalysts for purification of ethylene streams typically cites the ruthenium catalysts as examples of ineffective catalysts for such applications. For example, in U.S. Patent 4,299,800, a catalyst comprising 0.5 wt. % ruthenium on alumina was evaluated for oxygen removal from a feed stream containing ethylene. At low temperatures (50 °C), oxygen removal was low and ethylene conversion was essentially non-detectable. However, at higher temperatures (200°C), oxygen removal reached 99.4%, but with concomitant ethylene conversion (loss) of 11.2%, as compared to less than 5% ethylene conversion when using silver, gold or vanadium on alumina.

Thus, there is a need for a process for removing oxygen, acetylenes, and nitrogen oxides from off-gas streams wherein the ethylene is not converted to lower value hydrocarbons during the purification process and wherein the purified ethylene-containing gas stream comprises less than about 1 ppm each of acetylenes, nitrogen oxides and oxygen.

### DISCLOSURE OF THE INVENTION

The invention provides method for purifying ethylene from an ethylene-comprising gas stream obtained from steam cracking or catalytic cracking processes, which ethylene-comprising gas stream further comprises acetylene, methylacetylene, oxygen and nitrogen oxides, the method comprising contacting the ethylene-comprising gas stream with a supported ruthenium catalyst comprising between 0.01 wt. % to 5 wt. % ruthenium, wherein the ruthenium is supported on alumina, wherein the supported ruthenium catalyst is heated to a temperature of at least 120 °C before making contact with the gas stream and maintained at a temperature of 120 °C to 300 °C, wherein the hydrogen partial pressure is held between 0.10 MPa and 1 MPa with a gas hourly space velocity of 1000 hr⁻¹ to 5,000 hr⁻¹ and wherein the gas stream is contacted with the supported catalyst until the purified gas stream comprises less than 1 ppm acetylene/methylacetylene, less than 1 ppm nitrogen oxides, and less than 1 ppm oxygen, wherein the gas stream is contacted with the catalyst in a continuous flow reactor.

### MODES FOR CARRYING OUT EMBODIMENTS OF THE INVENTION

The ethylene-containing feed stream may be the off-gas stream from any steam cracker, fluid catalytic cracker, or similar process. Typically, the off-gas stream includes hydrogen gas, carbon monoxide, oxygen, nitrogen oxides, ethane, ethylene and acetylene.

The ruthenium-based catalyst is distributed on alumina. Methods of preparing supported ruthenium catalysts are well-known in the art. Optionally, the catalyst may further include promoters, such as, silver, gold, copper, zinc, bismuth, lead or combinations thereof. In an exemplary embodiment, the catalyst comprises ruthenium distributed on an alumina support wherein the ruthenium is distributed on the support with a ruthenium salt solution.

In one embodiment, the catalyst comprises ruthenium distributed on an alumina support wherein the ruthenium is distributed on the support by impregnating an alumina support with a ruthenium salt solution. The catalyst comprises between 0.01 wt. % to 5 wt. % ruthenium. In other embodiments, the catalyst may comprise, or consist essentially of, 0.01 wt. % to 1 wt. % ruthenium, 0.1 wt. % to 0.5 wt. % ruthenium; 0.15 wt. % to 0.30 wt. % ruthenium, a minimum of 0.15 wt. % ruthenium, or a minimum of 0.3 wt. % ruthenium on alumina.

In an alternative embodiment, the catalyst comprises ruthenium distributed on an alumina support wherein the support has a BET surface area of at least 3 m²/g, and preferably has a BET surface area of about 3 m²/g to about 200 m²/g. Alternatively, the catalyst may comprise ruthenium on a low surface area support, ruthenium on a medium surface area support, or ruthenium on a high surface area support. In general, a support having a BET surface area between 1 m²/g -10 m²/g can be classified as a low surface area support. Medium surface area supports typically range between 10 m²/g - 60 m²/g while high surface area supports generally have a BET surface area greater than 60 m²/g. With respect to alumina, the ranges for low, medium and high surface areas are 1 m²/g -10 m²/g, 30 m²/g -60 m²/g , and greater than 60 m²/g, respectively. In another embodiment, the support is a medium surface area alumina support.

In other embodiments, the ruthenium is distributed on the outer layer of an alumina carrier in such a manner as to remain on the outer layer of the support. "Distributed on the outer layer" of the support means that the ruthenium may be located within any part of about a 300 µm distance that extends from the exterior surface of any part of the support towards the center of the support. The depth of the ruthenium distributed on the outer layer of the support may be constant or may vary, especially in places where pores are located on the exterior surface of the support.

The process comprises directly contacting the gas stream with the supported catalyst while maintaining a reaction temperature of at least 120°C until the acetylene content decreases to less than one (1) ppm and the nitrogen oxide content decreases to less than one (1) ppm and the oxygen content decreases to less than one (1) ppm. Alternatively, the process may comprise contacting the gas stream with the supported catalyst until the product stream is essentially free of impurities that may include acetylene, nitrogen oxides, oxygen and combinations thereof. In other embodiments, the removal of the acetylene, nitrogen oxide and oxygen contents may be either higher or lower, depending a number of factors including laws and regulations governing the operation of the FCC/DCC plant and/or the design of the plant.

The catalyst may be reduced or sulfided before use. The catalyst may be reduced after being loaded into the reactor and before introduction of the ethylene-containing gas stream by feeding hydrogen or a hydrogen-containing gas through the catalyst at a temperature of at least 120°C for at least one minute. The catalyst may be sulfided after being loaded into the reactor and before introduction of the ethylene-containing gas stream by feeding a sulfur-containing gas stream through the catalyst at a temperature of at least 150°C for at least one minute.

### Industrial applicability

The above described embodiments may be used to purify ethylene-containing feed streams from steam crackers, fluid catalytic crackers (FCC), or any type of hydrocarbon feed stream that includes hydrogen, carbon monoxide, oxygen, and acetylenes.

### Examples

As representative examples, several catalysts were acquired and evaluated for removal of impurities from an ethylene feed stream. These examples are presented to further explain the invention.

Catalyst samples evaluated:
Catalyst 1: Commercial Pd-based catalyst, OleMax 250; obtained from Süd-Chemie Inc., Louisville, Kentucky.
Catalyst 2: 0.15% ruthenium on a low surface area (3.6 m²/g) alumina carrier;
Catalyst 3: 0.15% ruthenium on a medium surface area (37 m²/g) alumina carrier;
Catalyst 4: 0.15% ruthenium on a high surface area (165 m²/g) alumina carrier;
Catalyst 5: 0.30% ruthenium on a high surface area (165 m²/g) alumina carrier.

### Catalyst samples evaluations:

The prepared catalysts are tested in a continuous flow reactor by loading approximately 50 cc of catalyst into the reactor and then feeding a contaminated ethylene-containing feed stream through the loaded catalyst. For testing purposes, in general, the reactor temperature is adjusted to a temperature of from 120°C to 300°C, the carbon monoxide content is held between about 0.05 wt. % and 5 wt. %, and the sulfur content is held below about 50 ppm. The hydrogen partial pressure is held between about 0.05 MPa and 2 MPa with a gas hourly space velocity of from about 500 hr⁻¹ to 10,000 hr⁻¹; more preferably, the hydrogen partial pressure is held between about 0.10 MPa and 1 MPa with a gas hourly space velocity of from 1000 hr⁻¹ to 5,000 hr⁻¹; and most preferably, the hydrogen partial pressure is held between 0.10 MPa and 0.3 MPa with a gas hourly space velocity of from 1500 hr⁻¹ to 3500 hr⁻¹ and with a hydrogen concentration from about 5% to about 15%.

Catalysts 1 - 4 are tested in the continuous flow reactor. Approximately 50 cc of catalyst is loaded into the reactor, the reactor temperature is adjusted to a predetermined temperature (as indicated in Table 1), and an ethylene-containing feed stream contaminated with oxygen, acetylene, and nitric oxide is fed through the reactor at a gas hourly space velocity of 2500 hr⁻¹ while the pressure is held at 1.9 MPa. Gas samples from an inlet and outlet reactor are analyzed using an on-line gas chromatograph and the findings are summarized in Table 1.

**Table 1**

| | Catalyst 1 | | Catalyst 2 | | Catalyst 3 | | Catalyst 4 | |
|---|---|---|---|---|---|---|---|---|
| Catalyst | Pd/ Al₂O₃ | | Ru/ Al₂O₃ | | Ru/ Al₂O₃ | | Ru/ Al₂O₃ | |
| Reactor Temp | 97.1 °C | | 200°C | | 217°C | | 197°C | |
| | Feed | Product | Feed | Product | Feed | Product | Feed | Produc |
| C₂H₄ (%) | 23.3 | 21.6 | 22.6 | 22.2 | 22.1 | 21.6 | 22.2 | 22.0 |
| H₂ (%) | 9.6 | 7.68 | 9.1 | 8.6 | 8.7 | 8.3 | 8.5 | 8.1 |
| C₂H₂ (ppm) | 406 | 0.4 | 680 | <0.5 | 656 | <0.5 | 668 | <0.5 |
| NO (ppm) | 0.413 | <0.010 | 0.62 | 0.01 | 0.707 | 0.015 | 0.584 | 0.017 |
| O₂ (ppm) | 3663 | 3561 | 2891 | 0.37 | 2787 | 0.54 | 2792 | 0.32 |
| CO (%) | 0.59 | 0.50 | 2.18 | 1.88 | 2.38 | 2.09 | 2.32 | 1.99 |
| C₂H₆, (ppm) | 60 | 27270 | 37 | 2638 | 34 | 1821 | 36 | 3197 |

As indicated in Table 1, the palladium catalyst and the ruthenium catalysts all effectively retain ethylene and hydrogen in the gas stream, although the ruthenium catalysts retain a higher relative percentage of these gases than is observed with the palladium catalyst. Further, the palladium catalyst and the ruthenium catalysts all effectively reduce the levels of acetylene and nitrogen oxides present in the feed stream. However, the ruthenium catalysts are significantly more effective at removing oxygen from the feed stream than the palladium catalyst. Also, most likely because the ruthenium catalysts are less active for hydrogenation of ethylene than the palladium catalyst, less ethane is produced when the ethylene-containing feed stream contacts the ruthenium catalysts than when the feed stream contacts the palladium catalyst.

Catalyst 5 is tested in the continuous flow reactor at various reactor temperatures, and with additional carbon monoxide or hydrogen sulfide in the feed stream. Approximately 50 cc of catalyst is loaded into the reactor, the reactor temperature is adjusted to a predetermined temperature (as indicated in Table 2), and an ethylene-containing feed stream contaminated with oxygen, acetylene and optionally, nitric oxide or carbon monoxide or hydrogen sulfide, is fed through the reactor at a gas hourly space velocity of 2500 hr⁻¹ while the pressure is held at 1.9 MPa. Gas samples from an inlet and outlet reactor are analyzed using an on-line gas chromatograph and the findings are summarized in Table 2.

**Table 2**

| Catalyst | Ru/Al₂O₃ | | | | | | | | | |
|---|---|---|---|---|---|---|---|---|---|---|
| Variable | Temperature | | | CO Addition | | | H₂S Addition | | | |
| React Temp | | 139°C | 154°C | | 162°C | 176°C | | 186°C | | |
| Gas Comp | Feed | Product | Product | Feed | Product | Product | Feed | Product | Product | Product |
| C₂H₄ (%) | 24.08 | 22.33 | 22.52 | 23.68 | 23.44 | 23.38 | 23.68 | 22.24 | 23.39 | 21.8 |
| H₂ (%) | 10.21 | 9.89 | 9.70 | 10.5 | 9.7 | 9.6 | 10.5 | 9.7 | 9.7 | 10.7 |
| CO (%) | 1.1 | 0.79 | 0.73 | 2.39 | 2.11 | 2.11 | 2.39 | 2.13 | 2.03 | 2.06 |
| H₂S (ppm) | - | - | - | - | - | - | - | 0 | 8 | 18 |
| C₂H₂ (ppm) | 469 | 2.5 | 0.4 | 501 | <0.5 | <0.5 | 501 | <0.5 | <0.5 | <0.5 |
| NO (ppm) | 0.5 | 0.016 | 0.008 | 0.959 | 0.020 | 0.023 | 0.959 | 0.013 | 0.017 | 0.017 |
| O₂ (ppm) | 2869 | 113 | 0.85 | 3181 | 43 | 0.54 | 3181 | 0.1 | 0.2 | 1.4 |
| C₂H₆ (ppm) | 21 | 1610 | 3850 | 17 | 1162 | 2742 | 17 | 2888 | 1440 | 830 |

As indicated in Table 2, even under adverse conditions of relatively high levels of CO and hydrogen sulfide, the ruthenium catalyst effectively retains ethylene and hydrogen in the gas stream. Further, the ruthenium catalyst effectively reduces the levels of acetylene and oxygen present in the feed stream, and produces relatively low quantities of unwanted ethane.

Thus, by contacting an ethylene-containing feed stream that further comprises hydrogen, carbon monoxide, oxygen, acetylene, and nitric oxide with a supported ruthenium catalyst, wherein the catalyst comprises between 0.01 wt. % to 5 wt. % ruthenium, in a continuous flow reactor with the catalyst held at a temperature of at least about 120°C, acetylenes, nitrogen oxides and oxygen can be removed from the gas stream with a minimal loss of ethylene.

## Claims

1. A method for purifying ethylene from an ethylene-comprising gas stream obtained from steam cracking or catalytic cracking processes, which ethylene-comprising gas stream further comprises acetylene, methylacetylene, oxygen and nitrogen oxides, the method comprising contacting the ethylene-comprising gas stream with a supported ruthenium catalyst comprising between 0.01 wt. % to 5 wt. % ruthenium, wherein the ruthenium is supported on alumina, wherein the supported ruthenium catalyst is heated to a temperature of at least 120 °C before making contact with the gas stream and maintained at a temperature of 120 °C to 300 °C, wherein the hydrogen partial pressure is held between 0.10 MPa and 1 MPa with a gas hourly space velocity of 1000 hr⁻¹ to 5,000 hr⁻¹ and wherein the gas stream is contacted with the supported catalyst until the purified gas stream comprises less than 1 ppm acetylene/methylacetylene, less than 1 ppm nitrogen oxides, and less than 1 ppm oxygen, wherein the gas stream is contacted with the catalyst in a continuous flow reactor.

2. The method of Claim 1 wherein the gas stream has a gas hourly space velocity of 1500 hr⁻¹ to 3500 hr⁻¹ with a hydrogen concentration of from 5% to 15%, and the hydrogen partial pressure is held between 0.10 MPa and 0.3 MPa.

3. The method of Claim 1 wherein the supported ruthenium catalyst further comprises a promoter.

4. The method of Claim 3 wherein the promoter is selected from the group consisting of silver, gold, copper, zinc, bismuth, lead or combinations thereof.

5. The method of Claim 1 wherein the supported ruthenium catalyst is reduced; or wherein the supported ruthenium catalyst is sulfided.

6. The method of Claim 1 wherein the method comprises:
(a) loading a continuous flow reactor with the ruthenium catalyst supported on alumina;
(b) heating the catalyst to a temperature of at least 120 °C in the reactor and maintaining the catalyst at a temperature of 120 °C to 300 °C;
(c) feeding the ethylene-comprising gas stream into the reactor under a hydrogen partial pressure of between 0.10 MPa and 1 MPa with a gas hourly space velocity of 1000 hr⁻¹ to 5,000 hr⁻¹ such that the gas stream is in contact with the catalyst; and
(d) removing the ethylene-comprising gas stream from contact with the catalyst.

7. The method of Claim 6 wherein the hydrogen partial pressure is held between about 0.10 MPa and 0.3 MPa with a gas hourly·space velocity of from about 1500 hr⁻¹ to 3500 hr⁻¹ and with a hydrogen concentration of from 5% to 15%.

8. The method of Claim 6 wherein the supported ruthenium catalyst further comprises a promoter selected from the group consisting of silver, gold, copper, zinc, bismuth, lead or combinations thereof.

9. The method of Claim 6 wherein after step (a) and before step (c) the catalyst is reduced, while in the reactor, in hydrogen or in a hydrogen-containing gas at a temperature of at least 100 °C for at least one minute; or wherein after step (a) and before step (c) the catalyst is sulfided, while in the reactor, in a sulfur-containing gas stream at a temperature of at least 150 °C for at least one minute.

## Patentansprüche

1. Verfahren zum Reinigen von Ethylen aus einem ethylenhaltigen Gasstrom, erhalten aus Dampfcrack- oder katalytischen Crackprozessen, wobei der ethylenhaltige Gasstrom ferner Acetylen, Methylacetylen, Sauerstoff und Stickstoffoxide umfasst, wobei das Verfahren Kontaktieren des ethylenhaltigen Gasstroms mit einem unterstützten Rutheniumkatalysator umfasst, der zwischen 0,01 Gew.-% und 5 Gew.-% Ruthenium umfasst, wobei das Ruthenium auf Aluminiumoxid getragen ist, wobei der unterstützte Rutheniumkatalysator auf eine Temperatur von mindestens 120 °C erhitzt wird, bevor ein Kontakt mit dem Gasstrom hergestellt wird, und bei einer Temperatur von 120 °C bis 300 °C gehalten wird, wobei der Wasserstoffpartialdruck zwischen 0,10 MPa und 1 MPa mit einer Gas-Stundenraumgeschwindigkeit von 1000 hr⁻¹ bis 5.000 hr⁻¹ gehalten wird und wobei der Gasstrom mit dem unterstützten Katalysator in Kontakt gebracht wird, bis der gereinigte Gasstrom weniger als 1 ppm Acetylen/Methylacetylen, weniger als 1 ppm Stickstoffoxide und weniger als 1 ppm Sauerstoff umfasst, wobei der Gasstrom mit dem Katalysator in einem Durchflussreaktor in Kontakt gebracht wird.

2. Verfahren nach Anspruch 1, wobei der Gasstrom eine Gas-Stundenraumgeschwindigkeit von 1500 hr⁻¹ bis 3500 hr⁻¹ mit einer Wasserstoffkonzentration von 5 % bis 15 % aufweist und der Wasserstoffpartialdruck zwischen 0,10 MPa und 0,3 MPa gehalten wird.

3. Verfahren nach Anspruch 1, wobei der unterstützte Rutheniumkatalysator ferner einen Promotor umfasst.

4. Verfahren nach Anspruch 3, wobei der Promotor ausgewählt ist aus der Gruppe bestehend aus Silber, Gold, Kupfer, Zink, Wismut, Blei oder Kombinationen davon.

5. Verfahren nach Anspruch 1, wobei der unterstützte Rutheniumkatalysator reduziert ist; oder wobei der unterstützte Rutheniumkatalysator sulfidiert ist.

6. Verfahren nach Anspruch 1, wobei das Verfahren Folgendes umfasst:
(a) Beladen eines Durchflussreaktors mit dem Rutheniumkatalysator, der auf Aluminiumoxid getragen ist;
(b) Erhitzen des Katalysators auf eine Temperatur von mindestens 120 °C im Reaktor und Beibehalten des Katalysators bei einer Temperatur von 120 °C bis 300 °C;
(c) Zuführen des ethylenhaltigen Gasstroms zum Reaktor unter einem Wasserstoffpartialdruck von zwischen 0,10 MPa und 1 MPa mit einer Gas-Stundenraumgeschwindigkeit von 1000 hr⁻¹ bis 5.000 hr⁻¹, sodass der Gasstrom in Kontakt mit dem Katalysator steht; und
(d) Entfernen des ethylenhaltigen Gasstroms vom Kontakt mit dem Katalysator

7. Verfahren nach Anspruch 6, wobei der Wasserstoffpartialdruck zwischen ungefähr 0,10 MPa und 0,3 MPa mit einer Gas-Stundenraumgeschwindigkeit von ungefähr 1500 hr⁻¹ bis 3500 hr⁻¹ und mit einer Wasserstoffkonzentration von 5 % bis 15 % gehalten wird.

8. Verfahren nach Anspruch 6, wobei der unterstützte Rutheniumkatalysator ferner einen Promotor umfasst, ausgewählt aus der Gruppe bestehend aus Silber, Gold, Kupfer, Zink, Wismut, Blei oder Kombinationen davon.

9. Verfahren nach Anspruch 6, wobei nach Schritt (a) und vor Schritt (c) der Katalysator, während er sich im Reaktor befindet, in Wasserstoff oder in einem wasserstoffhaltigen Gas bei einer Temperatur von mindestens 100 °C für mindestens eine Minute reduziert wird; oder wobei nach Schritt (a) und vor Schritt (c) der Katalysator, während er sich im Reaktor befindet, in einem schwefelhaltigen Gasstrom bei einer Temperatur von mindestens 150 °C für mindestens eine Minute sulfidiert wird.

## Revendications

1. Procédé de purification d'éthylène à partir d'un courant de gaz contenant de l'éthylène obtenu à partir des opérations de vapocraquage ou craquage catalytique, lequel courant de gaz comprenant en outre de l'acétylène, du méthylacétylène, de l'oxygène et des oxydes d'azote, le procédé comprenant la mise en contact du courant de gaz contenant de l'éthylène avec un catalyseur au ruthénium supporté comprenant entre 0,01% et 5% en poids de ruthénium, dans lequel le ruthénium est sur support d'alumine, dans lequel le catalyseur de ruthénium supporté est chauffé à une température d'au moins 120 °C avant d'entrer en contact avec le courant de gaz et il est maintenu à une température entre 120 et 300 °C, dans lequel la pression partielle d'hydrogène est conservée entre 0,10 et 1 MPa avec une vitesse spatiale horaire gazeuse entre 1 000 heures⁻¹ et 5 000 heures ⁻¹, et dans lequel le courant de gaz est mis en contact avec le catalyseur supporté jusqu'à ce que le courant de gaz purifié comprenne moins de 1 ppm d'acétylène/méthylacétylène, moins de 1 ppm d'oxydes d'azote et moins de 1 ppm d'oxygène, dans lequel le courant de gaz est mis en contact avec le catalyseur dans un réacteur à écoulement continu.

2. Procédé selon la revendication 1, dans lequel le courant de gaz a une vitesse spatiale horaire gazeuse entre 1 500 heures⁻¹ et 3500 heures⁻¹ avec une concentration en hydrogène de 5 à 15%, et la pression partielle d'hydrogène est maintenue entre 0,10 et 0,3 MPa.

3. Procédé selon la revendication 1, dans lequel le catalyseur au ruthénium supporté comprend en outre un promoteur.

4. Procédé selon la revendication 3, dans lequel le promoteur est choisi dans le groupe constitué de l'argent, de l'or, du cuivre, du zinc, du bismuth, du plomb ou de leurs combinaisons.

5. Procédé selon la revendication 1, dans lequel le catalyseur au ruthénium supporté est réduit ; ou dans lequel le catalyseur de ruthénium supporté est sulfuré.

6. Procédé selon la revendication 1, dans lequel le procédé comprend :
(a)le chargement d'un réacteur à écoulement continu avec le catalyseur au ruthénium sur support d'alumine ;
(b) le chauffage du catalyseur à une température d'au moins 120°C dans le réacteur et maintenir le catalyseur à une température entre 120 et 300 °C ;
(c) l'introduction du courant de gaz contenant de l'éthylène dans le réacteur sous une pression partielle d'hydrogène entre 0,10 et 1 MPa avec une vitesse spatiale horaire gazeuse entre 1 000 heures⁻¹ et 5 000 heures⁻¹ de sorte que le courant de gaz soit en contact avec le catalyseur ; et
(d) la mise hors contact du courant de gaz contenant de l'éthylène du catalyseur.

7. Procédé selon la revendication 6, dans lequel la pression partielle d'hydrogène est maintenue entre environ 0,10 et 0,3 MPa avec une vitesse spatiale horaire gazeuse d'environ 1 500 heures⁻¹ à 3 500 heures⁻¹ et avec une concentration d'hydrogène de 5 à 15%.

8. Procédé selon la revendication 6, dans lequel le catalyseur au ruthénium supporté comprend en outre un promoteur choisi dans le groupe constitué de l'argent, de l'or, du cuivre, du zinc, du bismuth, du plomb ou de leurs combinaisons.

9. Procédé selon la revendication 6, dans lequel, après l'étape (a) et avant l'étape (c), le catalyseur est réduit, pendant qu'il est dans le réacteur, dans l'hydrogène ou dans un gaz contenant de l'hydrogène à une température d'au moins 100 °C pendant au moins une minute ; ou dans lequel, après l'étape (a) et avant l'étape (c), le catalyseur est sulfuré, pendant qu'il est dans le réacteur, dans le courant de gaz contenant du soufre à une température d'au moins 150 °C pendant au moins une minute.
